# EUROPEAN PATENT SPECIFICATION

(11) **EP 3 223 917 B1**
(45) Date of publication and mention of the grant of the patent: **25.03.2020**
(21) Application number: 15805290.2
(22) Date of filing: 17.11.2015
(51) Int. Cl.: A62B 18/08

(54) **AIR-FED VISOR**
LUFTANGESTRÖMTER VISOR
VISIÈRE À ALIMENTATION EN AIR

(30) Priority: 28.11.2014 GB 201421156
(43) Date of publication of application: 04.10.2017
(73) Proprietor: Carlisle Fluid Technologies, Inc., Charlotte NC 28277 (US)
(72) Inventor: FERGUSON, Paul Gregory, Wirral Merseyside CH60 9HG (GB)
(74) Representative: Somervell, Thomas Richard
(86) International application number: PCT/IB2015/058890
(87) International publication number: WO 2016/083942

(56) References cited:
- DE-A1- 2 135 928
- US-A- 3 502 074
- US-A- 3 822 698

## Description

The present invention relates to an air-fed visor, and more particularly to a visor used for spray painting applications to protect a wearer of the visor from airborne contaminants.

Such visors are known, which include a visor assembly having a pliable mask that surrounds the wearer's face, forming enough of a seal against the face so that ambient air is not drawn past it when the wearer breathes in. A breathing air tube is held in place between the mask and the visor and has a row of holes through which the breathing air is provided into the space between the mask and visor. Problems with this arrangement are that the holes limit the direction and area of air distribution and can cause user discomfort, especially if the air is poorly directed, for example if jets of air from the holes blow onto the wearer's face.

It is an object of the present invention to provide an improved air-fed visor.

US 3,502,074 discloses an air-fed visor comprising features of the preamble of claim 1.

According to the present invention there is provided an air-fed visor as set forth in claim 1. Embodiments comprise a mask, at least part of which comprises a foam material. An air tube is provided for delivering air to the visor. An air distribution rose is disposed in the foam mask. The rose comprises an air inlet connected to the air tube and an air distribution chamber for distributing air from the air inlet into the foam material.

The foam is formed of a foam material having a thickness between an inner surface and an outer surface of the mask, and wherein the air distribution chamber comprises a plurality of outlets distributing air into the foam material in a direction substantially parallel to the inner and outer surfaces. The inner surface of the foam material of the mask is preferably permeable to air and the outer surface of the mask is preferably impermeable to air. The outlets may be distributed uniformly around the air distribution chamber. Alternatively, the outlets may be distributed non-uniformly around the air distribution chamber. The outlets may be distributed along one side of the air distribution chamber.

The rose may have an inner plate disposed on the inner surface of the foam material of the mask. The rose may have an outer plate disposed on the outer surface of the foam material of the mask. The inner plate and the outer plate may be inter-connected with each other across a thickness of the foam material of the mask. The inner plate and/or the outer plate may comprise a chamber wall extending from the plate across the thickness of the foam material of the mask, the chamber wall defining a perimeter of the air distribution chamber. The chamber wall may have apertures defining outlets of the air distribution chamber. Both the inner plate and the outer plate may comprise chamber walls each of which comprises corresponding castellations, whereby when the plates are interconnected, the castellations overlap to provide air outlets through the chamber walls. The inner plate and the outer plate may be inter-connected by a push-fit connection means. Alternatively, the inner plate and the outer plate may be inter-connected by means of fasteners such as screws. The inner plate and/or the outer plate may comprise teeth for providing a grip to prevent slippage of the rose relative to foam material of the mask. The air inlet may comprise an inlet pipe on the outer plate.

It is an advantage that the rose provides the breathing air so that this is more uniformly distributed and diffused through the foam of the mask.

Embodiments of the invention will now be described with reference to the following drawings.
Figure 1 is an illustration of an air-fed visor.
Figure 2 is an illustration of an embodiment of an air distribution rose of the visor of Figure 1.
Figures 3a and 3b illustrate two components that make up the air distribution rose of Figure 2
Figure 4 is an illustration showing a cross-section of through the air distribution rose and foam mask of the visor of Figure 1.
Figure 5 is an illustration of another embodiment of an air distribution rose of the visor of Figure 1.
Figures 6a and 6b illustrate two components that make up the air distribution rose of Figure 5.

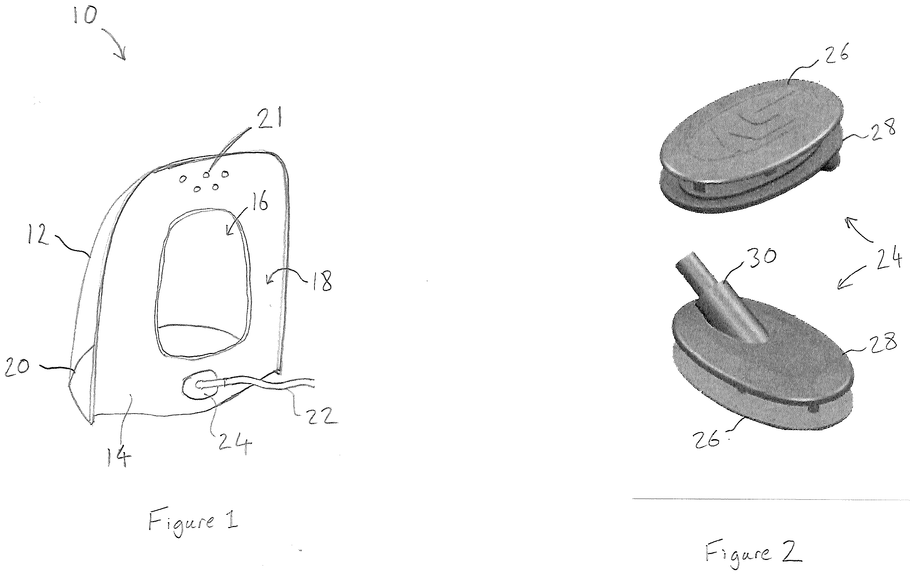

Figure 1 illustrates an air-fed visor 10. The visor 10 could be attached to headgear, such as a helmet, or could have an arrangement of straps for attaching to a wearer's head - these features are not shown. The visor has a transparent front panel 12, typically formed of cellulose acetate or a polymer or reinforced polymer material - alternatives could be cellulose propionate, PETG, polycarbonate, PVC, or triacetate. A mask 14, formed of a pliable material, at least part of which is a porous foam material such as open cell polyurethane (any open cell structure may be used) is joined at its perimeter to the visor panel 12, and has an opening 16 which has a size and shape to fit around a wearer's face.

The mask 14 provides a seal against the face for preventing, or restricting passage of ambient air. The foam material of the mask 14 is preferably laminated on both sides with elasticated nylon. One side is processed to provide an inner surface (not visible), which is permeable to air, and the other side to provide outer surface 18 (the visible surface in Figure 1), which is impermeable to air. Although not shown in Figure 1, the mask 14 is joined to the front lower rim 20 of the visor panel by the foam material or by other material, so that when the visor 10 is being worn, the space between the mask 14/wearer's face and the visor panel 12 is an enclosed space.

A breathing air tube 22 is connected to an air distribution rose 24 so that a supply of clean breathing air can be provided through the mask 14 into the sealed space. There are holes 21 cut in the foam and covered by a permeable material for the wearer's breath to escape when breathing out. As shown, these are preferably at the top of the mask 14 to encourage the flow from the breathing air tube 22 by the mouth, up across the face. There is also likely to be some leakage around the face as the seal between the mask and the wearer's face will not be perfect, as well as a small inward leakage of ambient air.

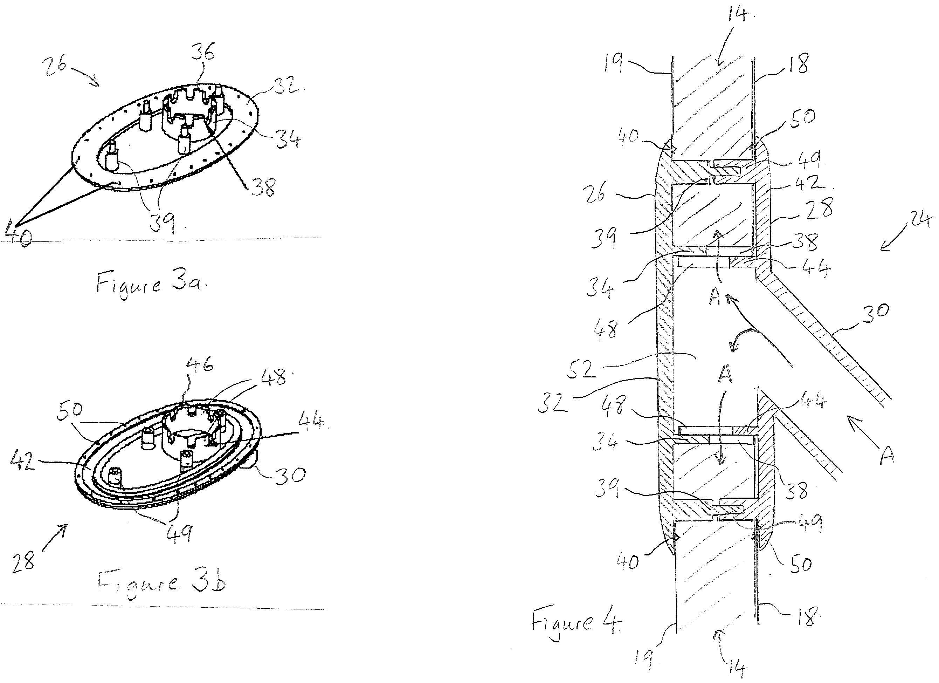

Figure 2 shows more detail of the air distribution rose 24 from two angles. The rose 24 is formed of two parts, an inner part 26 and an outer part 28 that will be described in more detail with reference to Figures 3a, 3b and 4 below. The upper illustration in Figure 2 shows the rose from the front side (i.e. the side that is exposed on the inner surface of the mask 14 of Figure 1), while the lower illustration shows the rose from the rear, or outer side. The rose 24 has an inlet pipe 30 for connecting to the breathing tube 22 of Figure 1.

Figure 3a shows the inner part 26 of the air distribution rose 24. The inner part 26 is typically formed of a rigid plastics material and comprises a plate 32. A first air chamber wall 34 extends from one side of the plate 32 to a rim 36, which has castellations 38. Also extending from the same side of the plate 32 are push-fit connectors 39 and a series of gripping teeth 40 around the outer region of the plate 32.

Figure 3b shows the outer part 28 of the air distribution rose 24. The outer part 28 is typically formed of a rigid plastics material and comprises a plate 42. A second air chamber wall 44 extends from one side of the plate 42 to a rim 46, which has castellations 48. The castellations 48 are positioned so as to coincide with positions of the castellation 38 on the inner part 26 when the two parts are connected. Also extending from the same side of the plate 42 are push-fit connectors 49, which compliment the push-fit connectors 39 on the inner part 26, and a series of gripping teeth 50 around the outer region of the plate 42.

Figure 4 is a cross-section illustrating the rose 24, when the inner part 26 and outer part 28 are connected together with the foam material mask 14 between them. The push-fit connectors 39 of the inner part are aligned with the complimentary push-fit connectors 49 of the outer part, thereby forming a push-fit connection to hold the inner part 26 and outer part 28 together. It will be appreciated that screws or other types of connection could be employed instead of push-fit connectors. The first air chamber wall 34 and second air chamber wall 44 are sized to provide an interference fit (in this example the first air chamber wall 34 on the inner part 26 surrounds the second air chamber wall 44 on the outer part 28, but these could equally well be the other way round). The first and second air chamber walls 34, 44 are thereby pushed tightly against each other forming an air chamber 52 within. The air inlet pipe 30 opens directly into the air chamber 52.

When the inner part 24 is connected to the outer part 26 the respective plates 32, 42 are separated by a distance that is slightly less than the thickness of the foam material of the mask 14, such that the foam material is trapped and gently squeezed between them. The gripping teeth 40, 50 on the inner and outer parts 26, 28 ensure that the foam material is retained and does not slip out from between the plates 32, 42.

With the inner and outer parts 26, 28 of the rose connected as shown in Figure 4, the castellations 38 on the first air chamber wall and the castellations 48 on the second air chamber wall overlap. In other words, one or other, or both, the sets of castellations extend over more than half of the distance separating the plates 32, 42 so that there are gaps through which air supplied to the chamber 52 can pass into the porous foam material of the mask 14, as indicated by the arrows A in figure 4.

As shown in Figure 4, the air distribution rose 24 is fixed into the mask 14 such that the inner part 26 is on the inner, air-permeable side 19 of the foam material, while the outer part 28 is on the outer impermeable side 18. Thus all the breathing air that is supplied to the air distribution rose 24 passes through into to the foam material of mask 14, as shown by the arrows A, and then permeates through the foam material and out through surface 19 into the space between the mask 14 and visor 12 (as shown in Figure 1).

It will be appreciated that the precise shape of the air inlet rose 24 may be varied and does not have to be an oval shape as shown. Similarly, the air chamber 52 does not have to be circular, but may be of any suitable shape as required. Also, the air outlets from the air chamber (resulting from the overlapping castellations 38, 48 in the depicted embodiment) are shown to be uniformly disposed around the air chamber. This may be preferred in order to provide a dispersed and uniform distribution of breathing air. However, other arrangements could be used in order to provide a different air distribution.

The provision of the castellations 38, 48 is a convenient way of providing outlet openings, particularly in a construction that is formed from moulded plastics parts. However, other ways may be employed to provide outlet openings - for example holes or slots could be formed in the chamber walls. Also, the chamber 52 could be formed with a single wall extending from one or other of the plates 32, 42, particularly if the inner and outer parts 26, 28 of the air distribution rose 24 are to be connected by means other than a push-fit connection.

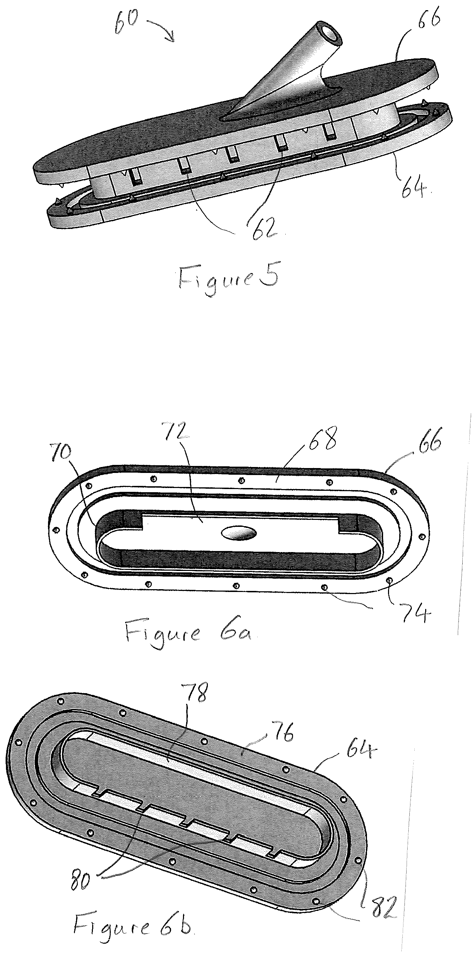

Figure 5 illustrates another embodiment of an air distribution rose 60 having a different shape and air distribution arrangement to that of the rose depicted in Figures 1 to 4. In this embodiment the rose 60 has a more elongated shape, and has air outlets 62 along one side only. As with the embodiment of Figures 1 to 4, the air distribution rose 60 is formed of two parts, an inner part 64 and an outer part 66.

As shown in Figures 6a, the outer part 66 of the air distribution rose 60, which is typically formed of a rigid plastics material, has a plate 68. An air chamber wall 70 extends from one side of the plate 68. In this case the air chamber wall 70 has a gap 72 along one side. A series of gripping teeth 74 are formed around the outer region of the plate 66.

As shown in Figure 6b, the inner part 64, also typically formed of a rigid plastics material, has a plate 76. A second air chamber wall 78 extends from one side of the plate 76 to a rim 78, which has castellations 80 on one side only. The castellations 80 are positioned to coincide with location of the gap 72 on the outer part 66 when the two parts are connected. The inner part 64 also includes a series of gripping teeth 82 around the outer region of the plate 42.

The air distribution rose 60, is assembled into a mask 14 in a similar way to the air distribution rose 24 as shown in Figure 4. In this case the openings for air from the central chamber are through the gaps between the castellations 80 along the one side of the rose 60. Thus, in this case breathing air is distributed into the foam material of the mask along only one side of the rose 60. This may be beneficial in directing air over the wearer's face so as to be most effective against the effects of any inward leakage. The porous foam material also distributes the flow or air so that the wearer does not suffer the discomfort of a draft air being blown onto the face.

The embodiments of the invention described above provide a simple and effective way of distributing breathing air into an air-fed visor, which overcome limitations in the prior art, including enabling a more diffuse distribution of air and the ability to direct the breathing air in a particular direction thereby improving the comfort of the wearer of the visor.

## Claims

1. An air-fed visor (10) comprising:
a mask (14) at least part of which comprises a foam material having a thickness between an inner surface and an outer surface (18) of the mask (14);
an air tube (22) for delivering air to the visor (10); and
a rose (24) disposed in the foam material of the mask (14), wherein the rose (24) comprises an air inlet (30) connected to the tube (22) and an air distribution chamber (52) for distributing air from the air inlet (30) into the foam material, the air distribution chamber (52) comprising a plurality of outlets (62) distributing air into the foam material,
**characterised in that** the outlets of the air distribution chamber are arranged to distribute the air in a direction substantially parallel to the inner and outer (18) surfaces.

2. The air-fed visor of claim 1, wherein the foam material of the mask (14) comprises an inner surface that is permeable to air.

3. The air-fed visor of claim 2, wherein the outer surface (18) of the mask (14) is impermeable to air.

4. The air-fed visor of claim 2 or claim 3, wherein the outlets (62) are distributed uniformly around the air distribution chamber (52).

5. The air-fed visor of claim 2 or claim 3, wherein the outlets (62) are distributed non-uniformly around the air distribution chamber (52), optionally wherein the outlets (62) are distributed along one side of the air distribution chamber (52).

6. The air-fed visor of any preceding claim, wherein the rose (24) has an inner plate (32) disposed on the inner surface of the foam material of the mask (14).

7. The air fed visor of claim 6, wherein the rose (24) has an outer plate (42) disposed on the outer surface (18) of the foam material of the mask (14).

8. The air-fed visor of claim 7, wherein the inner plate (32) and the outer plate (42) are inter-connected with each other across a thickness of the foam material of the mask (14).

9. The air-fed visor of claim 8, wherein the inner plate (32) and/or the outer plate (42) comprises a chamber wall (34, 44) extending from the plate (26, 28) across the thickness of the foam material of the mask (14), the chamber wall (34, 44) defining a perimeter of the air distribution chamber (52).

10. The air-fed visor of claim 9, wherein the chamber wall (34, 44) has apertures defining outlets (62) of the air distribution chamber (52).

11. The air-fed visor of claim 9, wherein both the inner plate (32) and the outer plate (42) comprise chamber walls (34, 44) each of which comprises corresponding castellations (38, 48), whereby when the plates (32, 42) are interconnected, the castellations (38, 48) overlap to provide air outlets (62) through the chamber walls (34, 44).

12. The air-fed visor of any of claims 6 to 11 wherein the inner plate (32) and the outer plate (42) are inter-connected either:
by a push-fit connection means (39, 49); or
by means of fasteners such as screws.

13. The air-fed visor of any of claims 6 to 12 wherein the inner plate (32) and/or the outer plate (42) comprises teeth (40, 50) for providing a grip to prevent slippage of the rose (24) relative to foam material of the mask (14).

14. The air-fed visor of any of claims 7 to 13 wherein the air inlet (30) comprises an inlet pipe on the outer plate (42).

## Patentansprüche

1. Luftgespeistes Visier (10), Folgendes beinhaltend:
eine Maske (14), von welcher mindestens ein Abschnitt ein Schaummaterial beinhaltet, welches eine Dicke zwischen einer inneren Oberfläche und eine äußeren Oberfläche (18) der Maske (14) besitzt;
eine Luftröhre (22) zum Abgeben von Luft an das Visier (10); und
eine Rosette (24), welche in dem Schaummaterial der Maske (14) angeordnet ist, wobei die Rosette (24) einen Lufteinlass (30) beinhaltet, welcher mit der Röhre (22) verbunden ist, und eine Luftverteilungskammer (52) zum Verteilen von Luft von dem Lufteinlass (30) in das Schaumaterial, wobei die Luftverteilungskammer (52) eine Vielzahl von Auslässen (62) beinhaltet, welche Luft in das Schaumaterial verteilt,
**dadurch gekennzeichnet, dass** die Auslässe der Luftverteilungskammer angeordnet sind, um die Luft in einer im Wesentlichen zu der inneren und der äußeren Oberfläche (18) parallelen Richtung zu verteilen.

2. Luftgespeistes Visier nach Anspruch 1, bei welchem das Schaumaterial der Maske (14) eine innere Fläche beinhaltet, welche gegenüber Luft durchlässig ist.

3. Luftgespeistes Visier nach Anspruch 2, bei welchem die äußere Oberfläche (18) der Maske (14) gegenüber Luft undurchlässig ist.

4. Luftgespeistes Visier nach Anspruch 2 oder 3, bei welchem die Auslässe (62) einheitlich um die Luftverteilungskammer (52) verteilt sind.

5. Luftgespeistes Visier nach Anspruch 2 oder 3, bei welchem die Auslässe (62) nicht einheitlich rund um die Luftverteilungskammer (52) verteilt sind, wobei optionsweise die Auslässe (62) entlang einer Seite der Luftverteilungskammer (52) verteilt sind.

6. Luftgespeistes Visier nach einem der vorhergehenden Ansprüche, bei welchem die Rosette (24) eine innere Platte (32) besitzt, welche an der inneren Oberfläche des Schaumaterials der Maske (14) angeordnet ist.

7. Luftgespeistes Visier nach Anspruch 6, bei welchem die Rosette (24) eine äußere Platte (42) besitzt, welche an der äußeren Oberfläche des Schaumaterials (18) der Maske (14) angeordnet ist.

8. Luftgespeistes Visier nach Anspruch 7, bei welchem die innere Platte (32) und die äußere Platte (42) miteinander über die Dicke des Schaumaterials der Maske (14) verbunden sind.

9. Luftgespeistes Visier nach Anspruch 8, bei welchem die innere Platte (32) und/oder die äußere Platte (42) eine Kammerwand (34, 44) beinhaltet/beinhalten, welche sich von der Platte (26, 28) über die Dicke des Schaumaterials der Maske (14) erstreckt, wobei die Kammerwand (34, 44) einen Umfang der Luftverteilungskammer (52) definiert.

10. Luftgespeistes Visier nach Anspruch 9, bei welchem die Kammerwand (34, 44) Öffnungen besitzt, welche Auslässe (62) der Luftverteilungskammer (52) definieren.

11. Luftgespeistes Visier nach Anspruch 9, bei welchem gleichermaßen die innere Platte (32) und die äußere Platte (42) Wände (34, 44) beinhalten, wobei jede davon entsprechende Zinnenstrukturen (38, 48) beinhaltet, wodurch, wenn die Platten (32, 42) miteinander verbunden sind, die Zinnenstrukturen (38, 48) überlappen, um Luftauslässe (62) durch die Kammerwände (34, 44) bereitzustellen.

12. Luftgespeistes Visier nach einem der Ansprüche 6 bis 11, bei welchem die innere Platte (32) und die äußere Platte (42) entweder miteinander verbunden sind:
durch ein Steckverbindungsmittel (39, 49); oder
mithilfe von Befestigungselementen wie beispielsweise Schrauben.

13. Luftgespeistes Visier nach einem der Ansprüche 6 bis 12, bei welchem die innere Platte (32) und/oder die äußere Platte (42) Zähne (40, 50) zum Bereitstellen eines Grips zum Vermeiden von Schlupf der Rosette (24) in Bezug auf das Schaumaterial der Maske (14) beinhaltet/beinhalten.

14. Luftgespeistes Visier nach einem der Ansprüche 7 bis 13, bei welchem der Lufteinlass (30) ein Einlassröhrchen an der äußeren Platte (42) beinhaltet.

## Revendications

1. Visière à alimentation en air (10) comprenant :
un masque (14) dont au moins une partie comporte un matériau en mousse ayant une épaisseur entre une surface intérieure et une surface extérieure (18) du masque (14),
un tube d'air (22) destiné à délivrer de l'air à la visière (10), et
une rosette (24) disposée dans le matériau de mousse du masque (14), dans laquelle la rosette (24) comprend une entrée d'air (30) reliée au tube (22) et une chambre de distribution d'air (52) pour distribuer de l'air, depuis l'entrée d'air (30), dans le matériau en mousse, la chambre de distribution d'air (52) comprenant une pluralité de sorties (62) distribuant de l'air dans le matériau en mousse,
**caractérisée en ce que** les sorties de la chambre de distribution d'air sont agencées de manière à distribuer de l'air dans une direction sensiblement parallèle aux surfaces intérieure et extérieure (18).

2. Visière à alimentation en air selon la revendication 1, dans laquelle le matériau en mousse du masque (14) comprend une surface intérieure qui est perméable à l'air.

3. Visière à alimentation en air selon la revendication 2, dans laquelle la surface extérieure (18) du masque (14) est imperméable à l'air.

4. Visière à alimentation en air selon la revendication 2 ou 3, dans laquelle les sorties (62) sont réparties uniformément autour de la chambre de distribution d'air (52).

5. Visière à alimentation en air selon la revendication 2 ou 3, dans laquelle les sorties (62) sont réparties de façon non uniforme autour de la chambre de distribution d'air (52), facultativement dans laquelle les sorties (62) sont réparties le long d'un côté de la chambre de distribution d'air (52).

6. Visière à alimentation en air selon l'une quelconque des revendications précédentes, dans laquelle la rosette (24) comporte une plaque intérieure (32) disposé sur la surface intérieure du matériau en mousse du masque (14).

7. Visière à alimentation en air selon la revendication 6, dans laquelle la rosette (24) comporte une plaque extérieure (42) disposé sur la surface extérieure (18) du matériau en mousse du masque (14).

8. Visière à alimentation en air selon la revendication 7, dans laquelle la plaque intérieure (32) et la plaque extérieur (42) sont interconnectés l'un à l'autre à travers l'épaisseur du matériau en mousse du masque (14).

9. Visière à alimentation en air selon la revendication 8, dans laquelle la plaque intérieur (32) et/ou la plaque extérieure (42) comprend une paroi de chambre (34, 44) s'étendant de la plaque (26, 28) à travers l'épaisseur du matériau en mousse du masque (14), la paroi de chambre (34, 44) définissant un périmètre de la chambre de distribution d'air (52).

10. Visière à alimentation en air selon la revendication 9, dans laquelle la paroi de chambre (34, 44) comprend des ouvertures définissant des sorties (62) de la chambre de distribution d'air (52).

11. Visière à alimentation en air selon la revendication 9, dans laquelle la plaque intérieure (32) et la plaque extérieure (42) comprennent tous deux des parois de chambre (34, 44) dont chacune comprend des créneaux (38, 48) correspondants, au moyen de quoi, lorsque les plaques (32, 42) sont interconnectées, les créneaux (38, 48) se chevauchent pour fournir des sorties d'air (62) à travers les parois de chambre (34, 44).

12. Visière à alimentation en air selon l'une quelconque des revendications 6 à 11, dans laquelle la plaque intérieure (32) et la plaque extérieure (42) sont interconnectés :
par des moyens de connexion enfichables (39, 49), ou
par des moyens de fixation tels que des vis.

13. Visière à alimentation en air selon l'une quelconque des revendications 6 à 12, dans laquelle la plaque intérieure (32) et/ou la plaque extérieure (42) comprend des dents (40, 50) pour fournir une préhension afin d'empêcher un glissement de la rosette (24) par rapport au matériau en mousse du masque (14).

14. Visière à alimentation en air selon l'une quelconque des revendications 7 à 13, dans laquelle l'entrée d'air (30) comprend un conduit d'entrée sur la plaque extérieure (42).
